(19) Europäisches Patentamt
European Patent Office
Office européen des brevets

(11) Veröffentlichungsnummer : **0 060 962**
**B1**

(12) # EUROPÄISCHE PATENTSCHRIFT

(45) Veröffentlichungstag der Patentschrift : **07.08.85**

(51) Int. Cl.⁴ : **C 07 D405/06**, A 01 N 43/64// C07D317/22

(21) Anmeldenummer : **81810477.0**

(22) Anmeldetag : **04.12.81**

(54) **Mikrobizide Triazolylmethyldioxolane und deren Herstellung.**

(30) Priorität : **10.12.80 CH 9102/80**

(43) Veröffentlichungstag der Anmeldung : **29.09.82 Patentblatt 82/39**

(45) Bekanntmachung des Hinweises auf die Patenterteilung : **07.08.85 Patentblatt 85/32**

(84) Benannte Vertragsstaaten : **AT BE CH DE FR GB IT LI LU NL SE**

(56) Entgegenhaltungen :
**DE-A- 2 943 631**
**US-A- 4 101 664**
**US-A- 4 101 666**
**US-A- 4 259 505**
**US-A- 4 338 327**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Sturm, Elmar, Dr.**
**Klusstrasse 66**
**CH-4147 Aesch (CH)**

**EP 0 060 962 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft substituierte 2-tert. Butyl-2-(1H-1,2,4-triazolyl-methyl)-4-oxymethyl-1,3-dioxolane der Formel I sowie ihre pflanzenverträglichen Salze mit anorganischen oder organischen Säuren und ihre Metallkomplexen, die Herstellung solcher Verbindungen, ferner mikrobizide Mittel, die Verbindungen der Formel I als Wirkstoffe enthalten und die Verwendung von Verbindungen der Formel I zur Bekämpfung von Pflanzenkrankheiten.

1-(2-Aryl-1,3-dioxolan-2-ylmethyl)-1H-1,2,4-triazol-Derivate sind aus DE-A-2 940 133 bekannt.

Es werden hierin Verbindungen der Formel I umfasst

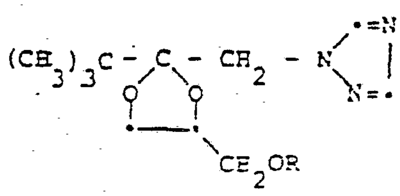

(I)

worin R einen gegebenenfalls ein- bis vierfach durch $C_1$-$C_4$-Alkyl, Halogen, Nitro, Cyano, Trifluormethyl, Phenyl oder $C_1$-$C_3$-Alkoxy substituierten Phenylrest oder 3,4-Methylendioxy-phenyl bedeutet, unter Einschluss ihrer pflanzenverträglichen Säureadditionssalze mit organischen und anorganischen Säuren sowie ihrer Metallkomplexe.

Unter Alkyl oder Alkylanteil eines anderen Substituenten sind je nach Zahl der angegebenen Kohlenstoffatome beispielsweise folgende Gruppen zu verstehen : Methyl, Ethyl, Propyl oder Butyl, sowie ihre Isomeren wie z. B. Isopropyl, Isobutyl, sec-Butyl oder tert-Butyl. Halogen steht für Fluor, Chlor, Brom oder Jod, vorzugsweise für Fluor, Chlor oder Brom.

Beispiele anorganischer Säuren sind Chlorwasserstoffsäure, Bromwasserstoffsäure, Jodwasserstoffsäure, Schwefelsäure, Phosphorsäure, phosphorige Säure und Salpetersäure.

Beispiele organischer Säuren sind Trifluoressigsäure, Trichloressigsäure, Benzolsulfonsäure und Methansulfonsäure.

Metallkomplexe der Formel I bestehen aus dem zugrundeliegenden organischen Molekül und einem anorganischen oder organischen Metallsalz, z. B. den Halogeniden, Nitraten, Sulfaten, Phosphaten, Tartraten usw. des Kupfers, Mangans, Nickels, Zinks und anderer Metalle. Dabei können die Metallkationen in den verschiedenen ihnen zukommenden Wertigkeiten vorliegen.

Verbindungen der Formel I zeigen ein sehr wertvolles Mikrobizid-Spektrum. Sie lassen sich z. B. gegen phytopathogene Mikroorganismen, insbesondere gegen pflanzenschädigende Pilze einsetzen.

Folgende Einzelverbindungen werden besonders bevorzugt :

a) 2-tert.-Butyl-2-(1H-1,2,4-triazol-1-yl)-methyl-4-(2,4-dichlorphenoxy)-methyl-1,3-dioxolan, unter Einschluss seiner Säureadditionssalze und Metallkomplexe.

b) 2-tert.-Butyl-2-(1H-1,2,4-triazol-1-yl)-methyl-4-(3-trifluormethylphenoxy)-methyl-1,3-dioxolan unter Einschluss seiner Säureadditionssalze und Metallkomplexe.

c) 2-tert.-butyl-2-(1H-1,2,4-triazol-1-yl)-methyl-4-(4-chlorphenoxy)-methyl-1,3-dioxolan, unter Einschluss seiner Säureadditionssalze und Metallkomplexe.

d) 2-tert.-butyl-2-(1H-1,2,4-triazol-1-yl)-methyl-4-(3,4-dichlorphenoxy)-methyl-1,3-dioxolan, unter Einschluss seiner Säureadditionssalze und Metallkomplexe.

Die Verbindungen der Formel I können, wie nachfolgend aufgeführt, hergestellt werden.

Ketale der Formel I können hergestellt werden durch Reaktion eines Triazols der Formel II.

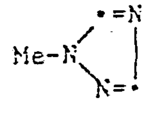

(II)

worin Me Wasserstoff oder bevorzugt ein Metallatom, insbesondere ein Alkalimetallatom darstellt, mit einer Verbindung der Formel III

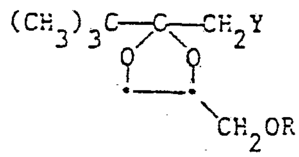

(III)

worin R die unter Formel I angegebenen Bedeutungen hat und Y für eine der üblichen Abgangsgruppen steht, beispielsweise für Halogen, insbesondere Chlor, Brom oder Jod, oder für Benzolsulfonyloxy, p-Tosyloxy, Trifluoracetyloxy oder bevorzugt Niederalkylsulfonyloxy wie Mesyloxy.

Die Reaktion von II mit III wird bevorzugt in einem relativ polaren, jedoch reaktionsinerten organischen Lösungsmittel durchgeführt, z. B. N,N-Dimethylformamid, N,N-Dimethylacetamid, Dimethylsulfoxid, Acetonitril, Hexamethylphosphorsäuretriamid, und anderen. Derartige Lösungsmittel können in Kombination mit anderen reaktionsinerten Lösungsmitteln, wie aliphatischen oder aromatischen Kohlenwasserstoffen, z. B. Benzol, Toluol, Xylol, Hexan, Petrolether, Chlorbenzol, Nitrobenzol u. a. verwendet werden.

Bedeutet Y Chlor oder Brom, so kann man zweckmässig Alkalijodid (wie NaJ oder KJ) zur Beschleunigung der Reaktion zusetzen. Erhöhte Temperaturen von 0 bis 220 °C, bevorzugt 80-170°, sind vorteilhaft. Zweckmässig wird das Reaktionsgemisch unter Rückfluss erhitzt.

Sobald in der Formel (II) Me für Wasserstoff steht, wird das Verfahren in Gegenwart einer Base durchgeführt. Beispiele solcher Basen sind anorganische Basen wie die Oxide, Hydroxide, Hybride, Carbonate und Hydrogencarbonate von Alkali und Erdalkalimetallen sowie z. B. tert.-Amine wie Triethylamin, Triethylendiamin, Pyridin, 4-Di-methylaminopyridin, 4-Pyrrolidylpyridin usw. oder Piperidin.

Bei diesem Herstellungsverfahren kann das Endprodukt der Formel I aus dem Reaktionsmedium isoliert und falls gewünscht, auf eine der allgemein üblichen Methoden gereinigt werden, z. B. durch Extraktion, Kristallisation, Chromatographie, Destillation usw.

Die Ausgangsketale der Formel III lassen sich z. B. entweder aus dem zugrundeliegenden Keton der Formel IV

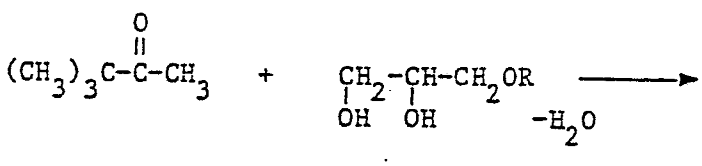

(III)

(IV) (V)

durch Reaktion mit dem Diol der Formel V in einem inerten Lösungsmittel, z. B. einem halogenierten Kohlenwasserstoff (wie Methylenchlorid, Ethylenchlorid, Chloroform, Tetrachlorkohlenstoff usw.) und gleichzeitige oder anschliessende Halogenierung, oder durch Reaktion des Ketons VI,

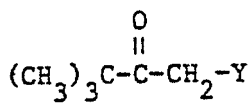

(VI)

worin Y die unter Formel III angegebene Bedeutung hat, mit dem Diol V in einem inerten Lösungsmittel gewinnen. Zur Beschleunigung der beiden Reaktionen ist ein Zusatz von p-Toluolsulfonsäure vorteilhaft.

Diese Ketalisierungs-Reaktionen können analog zu bereits bekannten Ketalisierungs-Reaktionen durchgeführt werden, beispielsweise analog zur Herstellung von 2-Brommethyl-2,4-diphenyl-1,3-dioxolan [Synthesis, *1974* (I), 23].

Bei der bevorzugten Ausführung der Ketalisierung werden beide Reaktionspartner mehrere Stunden zusammen mit einem Azeotrop-Bildner in einem der üblichen organischen Lösungsmittel unter Rückfluss erhitzt. Als Azeotrop-Bildner kommen z. B. Benzol, Toluol, Xylol, Chloroform oder Tetrachlor-Kohlenstoff in Frage. Hierbei arbeitet man beispielsweise in Gegenwart eines einfachen Alkohols, wie z. B. Ethanol, Propanol, Butanol, Pentanol usw., wobei zur Beschleunigung der Reaktion ein Zusatz einer starken Säure, wie z. B. p-Toluolsulfonsäure vorteilhaft sein kann. Verwendbare organische Lösungsmittel sind in diesem Fall z. B. aromatische Kohlenwasserstoffe, wie Benzol, Toluol, Xylol usw. und gesättigte Kohlenwasserstoffe, wie n-Hexan.

Das beschriebene Herstellungsverfahren ist ein Bestandteil der Erfindung.

Bei den beschriebenen Ketalisierungs-Reaktionen eines Ketons mit einem substituierten α,β-Diol vermögen Gemische von Diastereomeren des resultierenden Ketals zu entstehen. Entsprechend bilden sich aus den Ausgangsketonen im allgemeinen Diastereomerengemische der Endprodukte I. Die Verbindungen der Formel I können in nachfolgenden beiden diastereomeren Formen vorliegen :

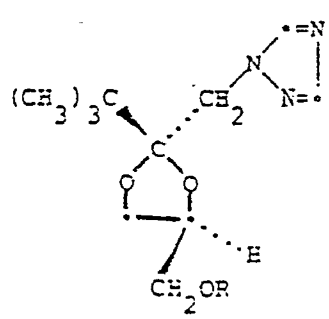

(Ia)

Die Konfiguration Ia soll hier und im folgenden als das « trans »-Isomere bezeichnet werden.

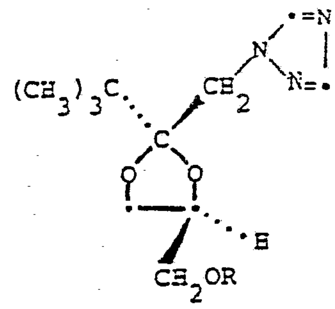

(Ib)

.... = hinter
— = in
▶— = vor der Zeichenebene

Die Konfiguration Ib soll entsprechend als das « Cis »-Isomere bezeichnet werden. Die Trennung der beiden Diastereomeren Ia und Ib kann beispielsweise durch fraktionierte Kristallisation oder durch Chromatographie (Dünn-, Dickschicht-, Säulenchromatographie, Flüssigkeitshochdruckchromatographie usw.) erfolgen. Die beiden Isomeren zeigen zum Teil unterschiedliche mikrobizide Wirkung. Im allgemeinen werden für praktische Zwecke die Diastereomerengemische verwendet. Die Charakterisierung der beiden Konfigurationen kann z. B. mit Hilfe NMR-spektroskopischer Methoden erfolgen. Die Erfindung bezieht sich auf sämtliche isomeren Verbindungen, ihre Salze und Metallkomplexe.

Die Ausgangsverbindung II, IV, V und VI sind bekannt und können nach an sich bekannten Methoden hergestellt werden.

1-(β-Aryl)-ethylimidazolylketale, worin Aryl für substituiertes Phenyl oder Naphthyl steht, werden in folgenden Referenzen als Fungizide und Bakterizide zitiert : US-PS : 3 575 999, 3 936 470, 4 101 664, 4 101 666, 4 156 008.

Es wurde überraschend gefunden, dass Verbindungen der Formel I ein für praktische Bedürfnisse sehr günstiges Mirkobizid-Spektrum aufweisen. Sie lassen sich beispielsweise zum Schutz von Kulturpflanzen verwenden.

Das Haupteinsatzgebiet von Verbindungen der Formel I liegt in der Bekämpfung von schädlichen Mikroorganismen, vor allem von phytopathogenen Pilzen. So besitzen die Verbindungen der Formel I eine für praktische Bedürfnisse sehr günstige kurative, präventive und systemische Wirkung zum Schutz von Kulturpflanzen ohne diese durch unerwünschte Nebenwirkungen zu beeinflussen. Kulturpflanzen seien im Rahmen vorliegender Erfindung beispielsweise : Getreide : (Weizen, Gerste, Roggen, Hafer, Reis) ; Rüben : (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) oder Pflanzen wie Mais, Tabak, Nüsse, Kaffee, Zuckerrohr, Tee, Weinreben, Hopfen, Bananen- und Naturkautschukgewächse sowie Zierpflanzen.

Mit den Wirkstoffen der Formel I können an Pflanzen oder an Pflanzenteilen (Früchte, Blüten, Laubwerk, Stengel, Knollen, Wurzeln) dieser und verwandter Nutzkulturen die auftretenden Mikroorganismen eingedämmt oder vernichtet werden, wobei auch später zuwachsende Pflanzenteile von derartigen Mikroorganismen verschont bleiben. Die Wirkstoffe sind gegen die den folgenden Klassen angehörenden phytopathogenen Pilze wirksam : Ascomycetes (z. B. Venturia, Erysiphaceae) ; Basidiomycetes wie vor allem Rostpilze (z. B. Puccinia) ; Fungi imperfecti (z. B. Moniliales u. a., Botrytis und die zur Familie der Dematiaceae gehörenden Cercospora-Erreger). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft somit ferner die Verwendung der Verbindungen der Formel I zur Bekämpfung phytopathogener Mikroorganismen bzw. zur präventiven Verhütung eines Befalls an Pflanzen.

Die erfinderischen Ketale der Formel I weisen gegenüber den zitierten Verbindungen ein zum Schutz von Kulturpflanzen verbessertes Mikrobizid-Spektrum auf und zeichnen sich bei den im Pflanzenschutz üblichen Aufwandmengen durch fehlende Phytotoxizität aus, so dass sie Kulturpflanzen vor schädlichen Mikroorganismen schützen, ohne sie dabei zu schädigen.

Zur Bekämpfung dieser Mikroorganismen können die Verbindungen der Formel I für sich allein oder zusammen mit geeigneten Trägern und/oder anderen Zuschlagstoffen verwendet werden. Geeignete Träger und Zuschlagstoffe können fest oder flüssig sein und entsprechen den in der Formulierungstechnik üblichen Stoffen wie z. B. natürlichen oder regenerierten mineralischen Stoffen, Lösungs-,

Dispergier-, Netz-, Haft-, Verdickungs-, Binde- oder Düngemitteln. Wirkstoffe der Formel I können auch im Gemisch mit z. B. pestiziden oder pflanzenwuschsverbessernden Präparaten verwendet werden.

Die nachfolgende, beispielshafte Aufzählung soll die Beschaffenheit solcher Mittel näher erläutern.

Der Gehalt an Wirkstoff in handelsfähigen Mitteln liegt zwischen 0,000 1 bis 90 %.

In den nachfolgenden Beispielen sind Temperaturen in Celsiusgraden angegeben. Teile und Prozentangeben beziehen sich stets auf das Gewicht.

Herstellungsbeispiele

Beispiel 1

a) Herstellung eines Ausgangsproduktes

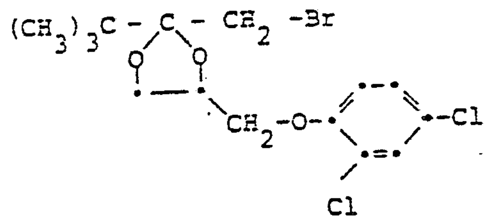

2-tert.-Butyl-2-brommethyl-4-(2,4-dichlorphenoxy)-methyl-1,3-dioxolan

27 g Brompinakolin und 37 g Glycerin-1-(2,4-dichlorphenyl)-ether werden unter Zusatz von 0,2 g p-Toluolsulfonsäure 48 Stunden in einem Gemisch aus 200 ml Toluol und 50 ml n-Butanol unter Verwendung eines Wasserabscheiders zum Sieden erhitzt. Die auf Raumtemperatur abgekühlte Reaktionslösung wird zweimal mit Natriumhydrogencarbonat und einmal mit Wasser gewaschen, über Natriumsulfat getrocknet und eingedampft. Das entstandene farblose Oel kristallisiert nach Zusatz von 10 ml Methanol.

Ausbeute 57 g Schmp. 80-83°.

b) Herstellung des Endproduktes

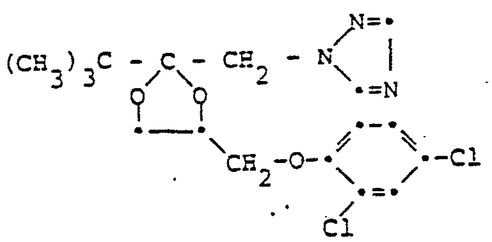

2-tert.-Butyl-2-(1H-1,2,4-triazol-1-yl)-methyl-4-(2,4-dichlorphenoxy)-methyl-1,3-dioxolan.

3,5 g 1,2,4-Triazol-Kaliumsalz werden in 100 ml absolutem Dimethylformamid auf 100° erhitzt. Zu der heissen Lösung lässt man 9 g 2-tert.-Butyl-2-bromethyl-4-(2,4-dichlorphenoxy)-methyl-1,3-dioxolan, gelöst in 50 ml absolutem Dimethylformamid, tropfen. Man rührt das Reaktionsgemisch anschliessend noch 24 Stunden bei 130°, entfernt das Lösungsmittel im Vakuum und extrahiert dem Rückstand mit Diethylether, wäscht die Etherphase mit Wasser und trocknet sie über Natriumsulfat. Nach dem Eindampfen erhält man 7 g eines viskosen Oels, das nach Zusatz von n-Hexan kristallisiert, wobei das cis-Isomere mit Smp. 113-114° entsteht. Das trans-Isomere mit Smp. 73-74° kann aus der Mutterlauge gewonnen werden.

Auf analoge Weise lassen sich folgende Endprodukte (falls nicht besonders vermerkt Diastereomerengemische mit unterschiedlichen, nicht näher untersuchten Mischungsverhältnissen) der Formel I herstellen :

(Siehe Tabelle 1 Seite 6 ff.)

Tabelle 1 : Verbindungen der Formel I

| Nr. | R | Salz bzw. Komplex | phys. Daten |
|---|---|---|---|
| 1 | $C_6H_5-$ | - | Smp. 80-82° |
| 2 | $2-CH_3-4-CH_3-C_6H_3-$ | - | Smp. 113-115° |
| 3 | 4-tert. $C_4H_9-C_6H_4-$ | - | Smp. 98-100° |
| 4 | $4-C_6H_5-C_6H_4-$ | - | Smp. 110-114° |
| 5 | $3-CF_3-C_6H_4-$(trans) | - | Smp. 96-97° |
| 6 | $3-CF_3-C_6H_4-$(cis) | - | viskoses Oel |
| 7 | $3-CF_3-C_6H_4-$(cis) | $CuCl_2$ | Smp. 228-230° |
| 8 | $4-F-C_6H_4-$ | - | Smp. 50-59° |
| 9 | $4-F-C_6H_4-$ | $HNO_3$ | Smp. 130-132° |
| 10 | $4-Cl-C_6H_4-$ | $HNO_3$ | Smp. 126-129° |
| 11 | $4-Br-C_6H_4-$ | - | Smp. 109-111° |
| 12 | $2-Cl-4-Cl-C_6H_3-$ *) | - | Smp. 69-97° |
| 13 | $2-Cl-4-Cl-C_6H_3-$ *) | HCl | Smp. 155-160° |
| 14 | $2-Cl-4-Cl-C_6H_3-$(trans) | - | Smp. 73-74° |
| 15 | $2-Cl-4-Cl-C_6H_3-$(cis) | - | Smp. 113-114° |
| 16 | $2-Cl-6-Cl-C_6H_3-$ | - | Smp. 78-81° |
| 17 | $2-Cl-5-Cl-C_6H_3-$ | - | Smp. 133-134° |
| 18 | $2-CH_3-4-Cl-C_6H_3-$ | - | viskoses Oel |
| 19 | $2-F-C_6H_4-$ | - | viskoses Oel |
| 20 | $3-F-C_6H_4-$ | - | hochviskos |
| 21 | $2-Cl-C_6H_4-$ | - | viskoses Oel |
| 22 | $3-Cl-C_6H_4-$ | - | |
| 23 | $3-Br-C_6H_4-$ | - | viskoses Oel |
| 24 | $2-Cl-3-Cl-C_6H_3-$ | - | |

Tabelle 1 (Fortsetzung)

| Nr. | R | Salz bzw. Komplex | phys. Daten |
|---|---|---|---|
| 25 | 3-Cl-5-Cl-$C_6H_3$- | - | viskoses Oel |
| 26 | 3-Cl-4-Cl-$C_6H_3$- | - | Smp. 86-91° |
| 27 | 3-$OCH_3$-$C_6H_4$- | - | viskoses Oel |
| 28 | 3-$CH_3$-4-Cl-$C_6H_3$- | - | viskoses Oel |
| 29 | 2-$CH_3$-4-Cl-6-Cl-$C_6H_2$- | - | viskoses Oel |
| 30 | 3,4-(-O-$CH_2$-O-)-$C_6H_3$- | - | viskoses Oel |
| 31 | 2-Br-4-Cl-$C_6H_3$ | - | viskoses Oel |
| 32 | 3-$C_2H_5$-$C_6H_4$ | - | viskoses Oel |
| 33 | 2-$C_2H_5$-$C_6H_4$ | - | viskoses Oel |
| 34 | 4-$C_2H_5$-$C_6H_4$ | - | Smp. 78-84° |
| 35 | 3-$CH_3$-4-Cl-5-$CH_3$-$C_6H_2$ | - | Smp.140-142° |
| 36 | 2-$OCH_3$-$C_6H_4$ | - | viskoses Oel |
| 37 | 2-$CH_3$-3-$CH_3$-$C_6H_3$ | - | viskoses Oel |
| 38 | 2-$CH_3$-3-$CH_3$-6-$CH_3$-$C_6H_2$ | - | viskoses Oel |
| 39 | 2-Br-$C_6H_4$ | - | viskoses Oel |
| 40 | 2-tert.-Butyl-5-$CH_3$-$C_6H_3$ | - | viskoses Oel |
| 41 | 2-$OCH_3$-6-$OCH_3$-$C_6H_3$ | - | viskoses Oel |
| 42 | 2-tert.-Butyl-$C_6H_4$ | - | viskoses Oel |

*) Diastereomerengemisch

Zur Applikation können die Verbindungen der Formel I in den folgenden Aufarbeitungsformen vorliegen.

## Formulierungsbeispiele

### Beispiel 2 : Feste Aufarbeitungsformen

Stäube- und Streumittel enthalten im allgemeinen bis zu 100 % des Wirkstoffes. Ein 5 %iges Stäubemittel kann beispielsweise aus 5 Teilen des Wirkstoffs und 95 Teilen eines Zuschlagstoffes wie

Talkum bestehen oder aus 5 Teilen Wirkstoff, 4 Teilen hochdisperser Kielsäure und 91 Teilen Talkum. Darüberhinaus sind weitere Gemische mit solchen und anderen in der Formulierungstechnik gebräuchlichen Trägermaterialien und Zuschlagstoffen denkbar. Bei der Herstellung dieser Stäubemittel werden die Wirkstoffe mit den Träger- und Zuschlagstoffen vermischt und vermahlen und können in dieser Form verstäubt werden.

Granulate wie Umhüllungsgranulate, Imprägniergranulate, Homogengranulate und Pellets [= Körner] enthalten üblicherweise 1 bis 80 % des Wirkstoffs. So kann sich ein 5 %iges Granulat z. B. aus 5 Teilen des Wirkstoffs, 0,25 Teilen expodiertem Pflanzenöl, 0,25 Teilen Cetylpolyglykolether, 3,50 Teilen Polyethylenglykol und 91 Teilen Kaolin (bevorzugte Korngrösse 0,3-0,8 mm) zusammensetzen. Man kann bei der Herstellung des Granulates wie folgt vorgehen :

Die Aktivsubstanz wird mit epoxidiertem Pflanzenöl vermischt und mit 6 Teilen Aceton gelöst, hierauf wird Polyethylenglykol und Cetylpolyglykolether zugesetzt. Die zo erhaltene Lösung wird auf Kaolin aufgesprüht, und anschliessend wird das Aceton im Vakuum verdampft. Ein derartiges Mikrogranulat wird vorteilhaft zur Bekämpfung von Bodenpilzen verwendet.

Beispiel 3 : Flüssige Aufarbeitungsformen

Man unterscheidet im allgemeinen zwischen Wirkstoffkonzentraten, die in Wasser dispergierbar oder löslich sind und Aerosolen. Zu den in Wasser dispergierbaren Wirkstoffkonzentraten zählen z. B. Spritzpulver (wettable powders) und Pasten, die üblicherweise in den Handelspackungen 25-90 % und in gebrauchsfertigen Lösungen 0,01 bis 15 % des Wirkstoffs enthalten. Emulsionskonzentrate enthalten 10 bis 50 % und Lösungskonzentrate enthalten in der gebrauchsfertigen Lösung 0,000 1 bis 20 % Aktivsubstanz. So kann ein 70 %iges Spritzpulver z. B. aus 70 Teilen des Wirkstoffs, 5 Teilen Natriumdibutylnaphthylsulfonat, dazu 3 Teilen Naphthalinsulfonsäuren — Phenolsulfonsäuren — Formaldehyd-Kondensat (im Mischverhältnis 3 : 2 : 1), 10 Teilen Kaolin und 12 Teilen Kreide, z. B. Champagne-Kreide zusammengesetzt sein. Ein 40 %-iges Spritzpulver kann z. B. aus folgenden Stoffen bestehen : 40 Teile Wirkstoff, 5 Teile Natrium-Ligninsulfonat, 1 Teil Natrium-Dibutylnaphthylsulfonat und 54 Teile Kieselsäure. Die Herstellung eines 25 %igen Spritzpulvers kann auf unterschiedliche Art erfolgen. So kann dieses sich z. B. zusammensetzen aus : 25 Teilen der Aktivsubstanz, 4,5 Teilen Calcium-Ligninsulfonat, 1,9 Teilen Kreide, [z. B. Champagne-Kreide] Hydroxyethylencellulose-Gemisch (1 : 1), 1,5 Teilen Natrium-Dibutylnaphthylsulfonat, 19,5 Teilen Kieselsäure, 19,5 Teilen Kreide, [z. B. Champagne-Kreide] und 28,1 Teilen Kaolin, Ein 25 %iges Spritzpulver kann z. B. auch bestehen aus 25 Teilen Wirkstoff, 2,5 Teilen Isooctylphenoxypolyoxyethylen-ethanol, 1,7 Teilen [Champagne]-Kreide/Hydroxyethylcellulosegemisch (1 : 1), 8,3 Teilen Natriumsilikat, 16,5 Teilen Kieselgur und 46 Teilen Kaolin. Ein 10 %iges Spritzpulver lässt sich z. B. herstellen aus 10 Teilen des Wirstoffes, 3 Teilen eines Gemisches aus Natriumsalzen von gesättigten Fettalkoholsulfonaten, 5 Teilen Naphthalinsulfonsäure/Formaldehyd-Kondensat und 82 Teilen Kaolin. Andere Spritzpulver können Gemische darstellen aus 5 bis 30 % der Aktivsubstanz zusammen mit 5 Teilen eines aufsaugenden Trägermaterials wie Kieselsäure, 55 bis 80 Teilen eines Trägermaterials wie Kaolin und eines Dispergiermittelgemisches, bestehend aus 5 Teilen Natrium-Arylsulfonates sowie aus 5 Teilen eines Alkylarylpolyglykolethers. Ein 25 %iges Emulsions-Konzentrat kann z. B. folgende emulgierbare Stoffe enthalten : 25 Teile des Wirkstoffs, 2,5 Teile epoxidiertes Pflanzenöl, 10 Teile eines Alkylarylsulfonat/Fettalkoholpolyglykol ethers-Gemisches, 5 Teile Dimethylformamid und 57,5 Teile Xylol.

Aus solchen Konzentraten können durch Verdünnen mit Wasser Emulsionen der gewünschten Anwendugskonzentration hergestellt werden, die besonders zur Blattapplikation geeignet sind. Darüberhinaus können weitere Spritzpulver mit anderen Mischungsverhältnissen oder anderen in der Formulierungstechnik gebräuchlichen Trägermaterialien und Zuschlagstoffen hergestellt werden. Die Wirkstoffe werden in geeigneten Mischern mit den genannten Zuschlagstoffen innig vermischt und auf entsprechenden Mühlen und Walzen vermahlen. Man erhält Spritzpulver von vorzüglicher Benetzbarkeit und Schwebefähigkeit, die sich mit Wasser zu Suspensionen der gewünschten Konzentration verdünnt und insbesondere zur Blattapplikation verwenden lassen. Solche Mittel gehören ebenfalls zur Erfindung.

Mittel, die nach der oben beschriebenen Art formuliert wurden und als Wirkungskomponente eine Verbindung der Formel I (z. B. Verbindung Nr. 1, 4, 5, 7, 9, 10, 11, 12, 13, 14, 26 oder 34) enthielten, liessen sicht mit sehr gutem Erfolg zur Bekämpfung von phytopathogenen Mikroorganismen einsetzen. Mit gleich gutem oder ähnlichem Erfolg können auch andere Verbindungen aus der Tabelle 1 eingesetzt werden.

Biologische Beispiele

Die in den nachfolgenden Beispielen verwendeten Spritzbrühen wurden, wie oben beschrieben, formuliert.

Beispiel 4 : Wirkung gegen Cercospora arachidicola auf Erdnuss-Pflanzen

3 Wochen alte Erdnusspflanzen wurden mit einer aus Spritzpulver des Wirkstoffes hergestellten Spritzbrühe (0,02 % Aktivsubstanz) besprüht. Nach ca. 12 Stunden wurden die behandelten Pflanzen mit

einer Konidiensuspension des Pilzes bestäubt. Die infizierten Pflanzen wurden dann für ca. 24 Std. bei 90 % relativer Luftfeuchtigkeit inkubiert und dann im Gewächshaus bei ca. 22 °C aufgestellt. Der Pilzbefall wurde nach 12 Tagen ausgewertet.

Im Vergleich zur unbehandelten Kontrolle zeigten Pflanzen, die mit Wirkstoffen der Formel I behandelt waren, einen geringen oder fast keinen Pilzbefall.

Die Verbindungen Nr. 10 und 13 verhüten den Pilzbefall auch noch in einer Konzentration von 0,002 %.

Beispiel 5 : Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,06 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt.

Die Beurteilung der Rostpustelentwicklung erfolgte 12 Tage nach der Infektion. Die Verbindungen Nr. 1, 4, 5, 11, 12, 26 und 34 hemmten den Rostpustelbefall auf weniger als 10 % im Vergleich zu unbehandelten Kontrollpflanzen (100 % Rostpustelbefall).

Die Verbindungen Nr. 4 und 12 verhüten den Pilzbefall auch noch in einer Konzentration von 0,002 %.

b) Systemische Wirkung

Zu Weizenpflanzen wurde 5 Tage nach der Aussaat eine aus Spritzpulver des Wirkstoffes hergestellte Spritzbrühe gegossen (0,006 % Aktivsubstanz bezogen auf das Bodenvolumen). Nach 3 Tagen wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion. Verbindungen der Formel I zeigten starke Wirkung. Die Verbindungen Nr. 1, 7 und 11 verhüteten die Ausbreitung der Krankheit vollständig.

Beispiel 6 : Residual protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt. Unter anderen verhüteten die Verbindungen Nr. 5, 11, 12 und 26 den Krankheitsbefall sogar noch in einer Konzentration von 0,02 %.

Beispiel 7 : Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellt Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt. In den Versuchen a) und b) zeigten die Verbindungen der Formel I volle Wirkung (Pilzbefall vollständig verhindert). Die Verbindungen 1, 5, 11, 12, 13, 14, 26 und 34 zeigten im Versuch a) sogar noch in einer Verdünnungskonzentration von 0,002 % volle Wirkung. Die Verbindungen 5, 11, 12 und 14 zeigten neben anderen diese Wirkung auch im Versuch b) in 0,002 %iger Konzentration.

Beispiel 8 : Wirkung gegen Botrytis cinerea an Apfelfrüchten

Künstlich verletzte Aepfel wurden behandelt, indem eine aus Spritzpulver der Wirksubstanz

hergestellte Spritzbrühe auf die Verletzungsstellen aufgetropft wurde. Die behandelten Früchte wurden anschliessend mit einer Sporensuspension von Botrytis cinerea inokuliert und während einer Woche bei einer hohen Luftfeuchtigkeit und ca. 20 °C inkubiert.

Bei der Auswertung wurden die angefaulten Verletzungsstellen gezählt und daraus die fungizide Wirkung der Testsubstanz abgeleitet. Unter anderen hemmten die Verbindungen Nr. 9 und 10 den Pilzbefall im Vergleich zu unbehandelten Kontrollfrüchten (100 % Befall) fast vollständig.

**Patentansprüche** (für die Vertragsstaaten : BE, CH, DE, FR, GB, IT, LI, LU, NL, SE)

1. Verbindungen der Formel I

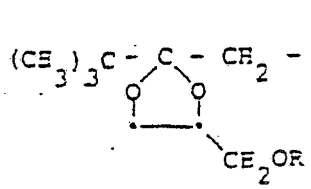

(I)

worin R einen gegebenenfalls ein- bis vierfach durch $C_1$-$C_4$-Alkyl, Halogen, Nitro, Cyano, Trifluormethyl, Phenyl oder $C_1$-$C_3$-Alkoxy substituierten Phenylrest oder 3,4-Methylendioxy-phenyl bedeutet, unter Einschluss ihrer pflanzenverträglichen Säureadditionssalze mit organischen und anorganischen Säuren sowie ihrer Metallkomplexe.

2. Die Verbindung 2-tert.-Butyl-2-(1H-1,2,4-triazol-1-yl)-methyl-4-(3-trifluormethyl-phenoxy)-methyl-1,3-dioxolan unter Einschluss seiner Säureadditionssalze und Metallkomplexe, nach Anspruch 1.

3. Die Verbindung 2-tert.-Butyl-2-(1H-1,2,4-triazol-1-yl)-methyl-4-(2,4-dichlorphenoxy)-methyl-1,3-dioxolan, unter Einschluss seiner Säureadditionssalze und Metallkomplexe, nach Anspruch 1.

4. Die Verbindung 2-tert.-Butyl-2-(1H-1,2,4-triazol-1-yl)-methyl-4-(4-chlorphenoxy)-methyl-1,3-dioxolan, unter Einschluss seiner Säureadditionssalze und Metallkomplexe, nach Anspruch 1.

5. Die Verbindung 2-tert.-Butyl-2-(1H-1,2,4-triazol-1-yl)-methyl-4-(3,4-dichlorphenoxy)-methyl-1,3-dioxolan, unter Einschluss seiner Säureadditionssalze und Metallkomplexe, nach Anspruch 1.

6. Verfahren zur Herstellung von Verbindungen der Formel I, gekennzeichnet durch Reaktion eines Triazols der Formel II

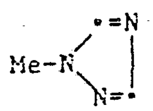

(II)

worin Me Wasserstoff oder ein Metallkation darstellt, mit einer Verbindung der Formel III

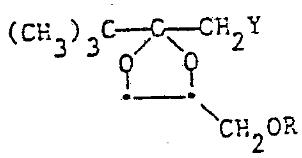

(III)

worin R die unter Formel I angegebenen Bedeutungen hat und Y für eine der üblichen Abgangsgruppen steht.

7. Schädlingsbekämpfungsmittel zur Bekämpfung von Mikroorganismen, dadurch gekennzeichnet, dass es als mindestens eine aktive Komponente eine Verbindung der Formel I gemäss Anspruch 1 zusammen mit einem oder mit mehreren geeigneten Trägerstoffen enthält.

8. Mittel zur Bekämpfung von Mikroorganismen gemäss Anspruch 7, dadurch gekennzeichnet, dass es als mindestens eine aktive Komponente eine Verbindung der Formel I gemäss einem der Ansprüche 2 bis 5 enthält.

9. Verwendung von Verbindungen der Formel I gemäss Anspruch 1 zur Bekämpfung und/oder zur präventiven Verhütung eines Befalls von Mikroorganismen.

10. Verwendung nach Anspruch 9 von Verbindungen der Formel I gemäss einem der Ansprüche 2 bis 5.

11. Verwendung gemäss einem der Ansprüche 9 oder 10, dadurch gekennzeichnet, dass es sich bei den Mikroorganismen um phytopathogene Pilze handelt.

**Patentansprüche** (für den Vertragsstaat AT)

1. Mittel zur Bekämpfung oder präventiven Verhütung eines Befalls von Mikroorganismen, dadurch gekennzeichnet, dass es als mindestens eine aktive Komponente eine Verbindung der Formel I,

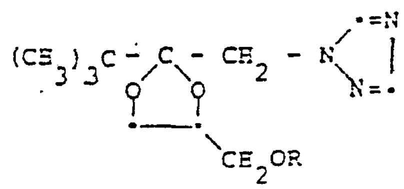

(I)

worin R einen gegebenenfalls ein- bis vierfach durch $C_1$-$C_4$-Alkyl, Halogen, Nitro, Cyano, Trifluormethyl, Phenyl oder $C_1$-$C_3$-Alkoxy substituierten Phenylrest oder 3,4-Methylendioxy-phenyl bedeutet, unter Einschluss ihrer pflanzenverträglichen Säureadditionssalze mit organischen und anorganischen Säuren sowie ihrer Metallkomplexe, zusammen mit einem oder mehreren geeigneten Trägerstoffen enthält.

2. Mittel nach Anspruch 1, dadurch gekennzeichnet, dass es 0,000 1 bis 90 Gewichtsprozent einer der in Anspruch 1 definierten Aktivkomponenten der Formel I enthält.

3. Mittel nach Anspruch 1, dadurch gekennzeichnet, dass es als aktive Komponente die Verbindung 2-tert.-Butyl-2-(1H-1,2,4-triazol-1-yl)-methyl-4-(3-trifluormethyl-phenoxy)-methyl-1,3-dioxolan unter Einschluss seiner Säureadditionssalze und Metallkomplexe enthält.

4. Mittel nach Anspruch 1, dadurch gekennzeichnet, dass es als aktive Komponente die Verbindung 2-tert.-Butyl-2-(1H-1,2,4-triazol-1-yl)-methyl-4-(2,4-dichlorphenoxy)-methyl-1,3-dioxolan, unter Einschluss seiner Säureadditionssalze und Metallkomplexe, enthält.

5. Mittel nach Anspruch 1, dadurch gekennzeichnet, dass es als aktive Komponente die Verbindung 2-tert.-Butyl-2-(1H-1,2,4-triazol-1-yl)-methyl-4-(4-chlorphenoxy)-methyl-1,3-dioxolan, unter Einschluss seiner Säureadditionssalze und Metallkomplexe, enthält.

6. Mittel nach Anspruch 1, dadurch gekennzeichnet, dass es als aktive Komponente die Verbindung 2-tert.-butyl-2-(1H-1,2,4-triazol-1-yl)-methyl-4-(3,4-dichlorphenoxy)-methyl-1,3-dioxolan, unter Einschluss seiner Säureadditionssalze und Metallkomplexe, enthält.

7. Verfahren zur Herstellung von Verbindungen der Formel I, gekennzeichnet durch Reaktion eines Triazols der Formel II

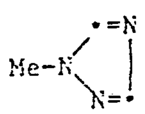

(II)

worin Me Wasserstoff oder ein Metallkation darstellt, mit einer Verbindung der Formel III

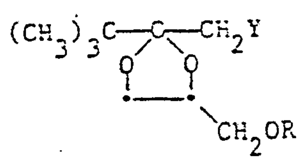

(III)

worin R die unter Formel I angegebenen Bedeutungen hat und Y für eine der üblichen Abgangsgruppen steht.

8. Verwendung einer der in Anspruch 1 definierten Verbindung der Formel I zur Bekämpfung und/oder zur präventiven Verhütung eines Befalls von Mikroorganismen.

9. Verwendung nach Anspruch 8 einer der in den Ansprüchen 3 bis 6 definierten Verbindungen der Formel I.

10. Verwendung gemäss einem der Ansprüche 8 oder 9, dadurch gekennzeichnet, dass es sich bei den Mikroorganismen um phytopathogene Pilze handelt.

**Claims** (for the Contracting States : BE, CH, DE, FR, GB, IT, LI, LU, NL, SE)

1. A compound of the formula I

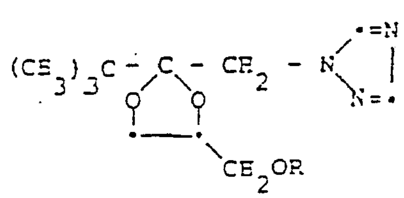

(I)

wherein R is a phenyl group which is unsubstituted or mono- to tetrasubstituted by $C_1$-$C_4$-alkyl, halogen, nitro, cyano, trifluoromethyl, phenyl or $C_1$-$C_3$-alkoxy, or it is 3,4-methylenedioxyphenyl, or an acid addition salt thereof with an organic or inorganic acid or a metal complex thereof, all tolerated by cultivated plants.

2. The compound 2-tert-butyl-2-(1H-1,2,4-triazol-1-yl)methyl-4-(3-trifluoromethylphenoxy)methyl-1,3-dioxolane, or an acid addition salt or a metal complex thereof, according to claim 1.

3. The compound 2-tert-butyl-2-(1H-1,2,4-triazol-1-yl)-methyl-4-(2,4-dichlorophenoxy)methyl-1,3-dioxolane, or an acid addition salt or a metal complex thereof, according to claim 1.

4. The compound 2-tert-butyl-2-(1H-1,2,4-triazol-1-yl)methyl-4-(4-chlorophenoxy)methyl-1,3-dioxolane, or an acid addition salt or a metal complex thereof, according to claim 1.

5. The compound 2-tert-butyl-2-(1H-1,2,4-triazol-1-yl)-methyl-4-(3,4-dichlorophenoxy)methyl-1,3-dioxolane, or an acid addition salt or a metal complex thereof, according to claim 1.

6. A process for the preparation of a compound of the formula I, which process comprises reacting a triazole of the formula II

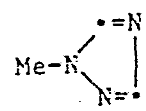

(II)

wherein Me is hydrogen or a metal cation, with a compound of the formula III

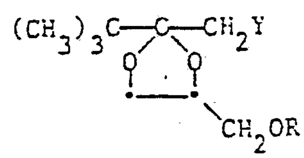

(III)

wherein R is as defined for formula I, and Y is one of the customary leaving groups.

7. A pesticidal composition for controlling microorganisms, which composition contains, as at least one active ingredient, a compound of the formula I according to claim 1, together with one or more suitable carriers.

8. A composition according to claim 7 for controlling microorganisms, which composition contains, as at least one active ingredient, a compound of the formula I as claimed in any one of claims 2 to 5.

9. A method of controlling and/or preventing infestation by microorganisms, which method comprises applying to the locus to be protected an effective amount of a compound of the formula I according to claim 1.

10. A method according to claim 9, which comprises applying an effective amount of a compound of the formula I as claimed in any one of claims 2 to 5.

11. A method according to either claim 9 or 10, wherein the microorganisms are phytopathogenic fungi.

**Claims** (for the Contracting State AT)

1. A composition for controlling or preventing infestation by microorganisms, which composition contains, as at least one active ingredient, a compound of the formula I

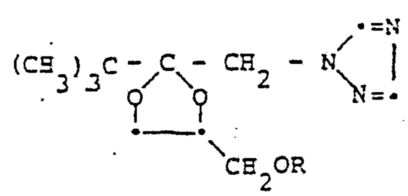

(I)

wherein R is a phenyl group which is unsubstituted or mono- to tetrasubstituted by $C_1$-$C_4$-alkyl, halogen, nitro, cyano, trifluoromethyl, phenyl or $C_1$-$C_3$-alkoxy, or it is 3,4-methylenedioxyphenyl, or an acid addition salt thereof with an organic or inorganic acid or a metal complex thereof, all tolerated by cultivated plants.

2. A composition according to claim 1, which contains 0.000 1 to 90 % by weight of an active ingredient of formula I as defined in claim 1.

3. A composition according to claim 1, which contains as active ingredient the compound 2-tert-butyl-2-(1H-1,2,4-triazol-1-yl)methyl-4-(3-trifluoromethylphenoxy)methyl-1,3-dioxolane, or an acid addition salt or a metal complex thereof.

4. A composition according to claim 1, which contains as active ingredient the compound 2-tert-butyl-2-(1H-1,2,4-triazol-1-yl)methyl-4-(2,4-dichlorophenoxy)methyl-1,3-dioxolane, or an acid addition salt or a metal complex thereof.

5. A composition according to claim 1, which contains as active ingredient the compound 2-tert-butyl-2-(1H-1,2,4-triazol-1-yl)methyl-4-(4-chlorophenoxy)methyl-1,3-dioxolane, or an acid addition salt or a metal complex thereof.

6. A composition according to claim 1, which contains as active ingredient the compound 2-tert-butyl-2-(1H-1,2,4-triazol-1-yl)methyl-4-(3,4-dichlorophenoxy)methyl-1,3-dioxolane, or an acid addition salt or a metal complex thereof.

7. A process for the preparation of a compound of the formula I, which process comprises reacting a triazole of the formula II

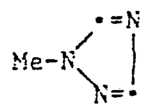

(II)

wherein Me is hydrogen or a metal cation, with a compound of the formula III

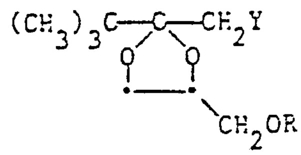

(III)

wherein R is as defined for formula I, and Y is one of the customary leaving groups.

8. A method of controlling and/or preventing infestation by microorganisms, which method comprises applying to the locus to be protected an effective amount of a compound of the formula I according to claim 1.

9. A method according to claim 8, which comprises applying an effective amount of a compound of the formula I as claimed in any one of claims 3 to 6.

10. A method according to either claim 8 or 9, wherein the microorganisms are phytopathogenic fungi.

**Revendications** (pour les Etats contractants : BE, CH, DE, FR, GB, IT, LI, LU, NL, SE)

1. Composés de formule I

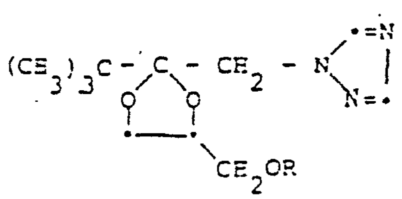

(I)

dans laquelle R représente un reste phényle éventuellement substitué une à quatre fois par des groupes alkyle en $C_1$-$C_4$, des halogènes, des groupes nitro, cyano, trifluorométhyle, phényle ou alcoxy en $C_1$-$C_3$, ou un groupe 3,4-méthylène-dioxyphényle, y compris leurs sels formés par addition avec des acides organiques et minéraux et qui sont tolérés par les végétaux, et leurs complexes métalliques.

2. Le composé 2-tert.-butyl-2-(1H-1,2,4-triazole-1-yl)-méthyl-4-(3-trifluorométhylphénoxy)-méthyl-1,3-dioxolanne, y compris ses sels formés par addition avec des acides et complexes métalliques, selon la revendication 1.

3. Le composé 2-tert.-butyl-2-(1H-1,2,4-triazole-1-yl)-méthyl-4-(2,4-dichlorophénoxy)-méthyl-1,3-dioxolanne, y compris ses sels formés par addition avec des acides et complexes métalliques, selon la revendication 1.

4. Le composé 2-tert.-butyl-2-(1H-1,2,4-triazole-1-yl)-méthyl-4-(4-chlorophénoxy)-méthyl-1,3-dioxolanne, y compris ses sels formés par addition avec des acides et complexes métalliques, selon la revendication 1.

5. Le composé 2-tert.-butyl-2-(1H-1,2,4-triazole-1-yl)-méthyl-4-(3,4-dichlorophénoxy)-méthyl-1,3-dioxolanne, y compris ses sels formés par addition avec des acides et complexes métalliques, selon la revendication 1.

6. Procédé de préparation des composés de formule I, caractérisé en ce que l'on fait réagir un triazole de formule II

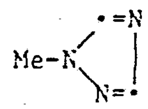

(II)

dans laquelle Me représente l'hydrogène ou un cation métallique, avec un composé de formule III

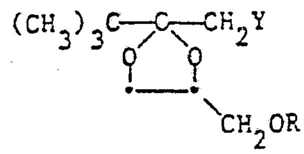

(III)

dans laquelle R a les significations indiquées en référence à la formule I et Y représente l'un des groupes éliminables usuels.

7. Produit pesticide pour la lutte contre les microorganismes, caractérisé en ce qu'il contient au moins un composant actif consistant en un composé de formule I selon la revendication 1, avec un ou plusieurs véhicules appropriés.

8. Produit pour combattre les microorganismes selon la revendication 7, caractérisé en ce qu'il contient au moins un composant actif consistant en un composé de formule I selon l'une des revendications 2 à 5.

9. Utilisation des composés de formule I selon la revendication 1, pour combattre et/ou prévenir une infestation par des microorganismes.

10. Utilisation selon la revendication 9, de composés de formule I selon l'une des revendications 2 à 5.

11. Utilisation selon l'une des revendications 9 ou 10, caractérisée en ce que les microorganismes sont des mycètes phytopathogènes.

**Revendications** (pour l'Etat contractant AT)

1. Produit pour combattre ou prévenir une infestation par des microorganismes, caractérisé en ce qu'il contient au moins un composant actif consistant en un composé de formule I

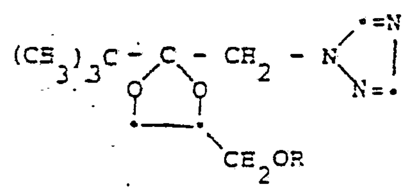

(I)

dans laquelle R représente un groupe phényle portant éventuellement un à quatre substituants alkyle en $C_1$-$C_4$, halogéno, nitro, cyano, trifluorométhyle, phényle ou alcoxy en $C_1$-$C_3$, ou un reste 3,4-méthylène-dioxy-phényle, y compris leurs sels formés par addition avec des acides organiques et minéraux qui sont tolérés par les végétaux et leurs complexes métalliques, avec un ou plusieurs véhicules appropriés.

2. Produit selon la revendication 1, caractérisé en ce qu'il contient de 0,000 1 à 90 % en poids de l'un des composants actifs de formule I définis dans la revendication 1.

3. Produit selon la revendication 1, caractérisé en ce qu'il contient en tant que composant actif le composé 2-tert.-butyl-2-(1H-1,2,4-triazole-1-yl)-méthyl-4-(3-trifluorométhylphénoxy)-méthyl-1,3-dioxolanne, y compris ses sels formés par addition avec des acides et complexes métalliques.

4. Produit selon la revendication 1, caractérisé en ce qu'il contient en tant que composant actif le composé 2-tert.-butyl-2-(1H-1,2,4-triazole-1-yl)-méthyl-4-(2,4-dichlorophénoxy)-1,3-dioxolanne, y compris ses sels formés par addition avec des acides et complexes métalliques.

5. Produit selon la revendication 1, caractérisé en ce qu'il contient en tant que composant actif le composé 2-tert.-butyl-2-(1H-1,2,4-triazole-1-yl)-méthyl-4-(4-chlorophénoxy)-méthyl-1,3-dioxolanne, y compris ses sels formés par addition avec des acides et complexes métalliques.

6. Produit selon la revendication 1, caractérisé en ce qu'il contient en tant que composant actif le composé 2-tert.-butyl-2-(1H-1,2,4-triazole-1-yl)-méthyl-4-(3,4-dichlorophénoxy)-méthyl-1,3-dioxolanne, y compris ses sels formés par addition avec des acides et complexes métalliques.

7. Procédé de préparation des composés de formule I, caractérisé en ce que l'on fait réagir un triazole de formule II

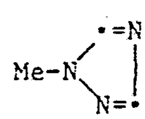

(II)

dans laquelle Me représente l'hydrogène ou un cation métallique, avec un composé de formule III

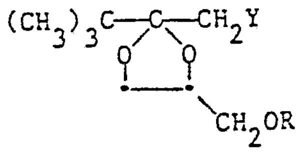

(III)

dans laquelle R a les significations indiquées en référence à la formule I et Y représente l'un des groupes éliminables usuels.

8. Utilisation d'un composé de formule I de la revendication 1 dans la lutte et/ou la prévention contre une infestation par des microorganismes.

9. Utilisation selon la revendication 8, de l'un des composés de formule I définis dans les revendications 3 à 6.

10. Utilisation selon l'une des revendications 8 ou 9, caractérisée en ce que les microorganismes sont des mycètes phytopathogènes.